# EUROPEAN PATENT APPLICATION

(11) **EP 4 180 012 A1**
(43) Date of publication of application: **17.05.2023**
(21) Application number: 21207932.1
(22) Date of filing: 12.11.2021
(51) Int. Cl.: A61F 13/15, A61F 13/496

(54) **A WASHABLE ABSORBENT GARMENT, AND METHOD AND APPARATUS FOR PRODUCING WASHABLE ABSORBENT GARMENTS**

(71) Applicant: Fameccanica.Data S.p.A., 66020 San Giovanni Teatino (CH) (IT)
(72) Inventor: LUPINETTI, Serafino, I-66020 San Giovanni Teatino (Chieti) (IT)
(74) Representative: Marchitelli, Mauro

(57) **Abstract**

A washable absorbent garment comprising a back layer (12), a washable absorbent core (28), and a top layer (30) which covers the washable absorbent core (28), wherein the top layer (30) and the washable absorbent core (28) are permanently fixed by ultrasonic, or mechanical welding and/or by glue in a crotch section (18) of the back layer (12).

## Description

### Field of the invention

The present invention relates to absorbent garment for the absorption and retention of liquids, and more particularly to washable and reusable absorbent garments.

The invention also relates to a method and apparatus for producing washable absorbent garments.

### Prior art

Various washable garments having a pant-like configuration and useful as incontinence garments, menstrual garments, training garments, diapers, and the like, are commercially available, as well as being disclosed in various patents.

For instance, absorbent garments are commercially available which are machine washable, and thus reusable, having an absorbent pad sewn into the crotch area.

US 3489149 discloses a menstrual panty having a pocket sewn into the crotch area, the pocket being used for receiving an absorbent disposable menstrual pad. Similarly, US 4352356 discloses a urinary incontinence panty having a pouch connected in the crotch area and adapted to receive an absorbent disposable urinary incontinence pad. Another sanitary panty garment with a pocket in the crotch area is disclosed in US 3613686.

Also, washable absorbent garments are known wherein the absorbent pad is permanently connected to the pant structure. For instance, GB2176692A discloses an absorbent sanitary garment made in the form of knitted polyester material which incorporates a non-removable pad made of brushed polyester which has the appearance of soft felt. The absorbent pad cannot be removed and is made of a material which can be frequently washed.

Prior art washable absorbent garments are manufactured by the same techniques uses for manufacturing textiles articles, typically by manually cutting and sewing fabric materials. In the prior art washable absorbent garments, a washable absorbent core is typically sewn to a fabric layer forming a pant-structure and opposite waist sections of the pant-structure are sewn to each other. The typical production rate of prior art washable absorbent garments is in the range of 1 piece/1'.

The main problem of the prior art washable absorbent garments is the high use of manual labor, and consequently the very low production rates and the high costs due to the high impact of manpower.

### Object and summary of the invention

The object of the present invention is to provide a washable absorbent garment which overcomes the problems of the prior art.

According to the present invention, this object is achieved by a washable absorbent garment having the features of claims 1.

According to another aspect, the invention relates to a method for producing washable absorbent garments having the features of claim 9 and to an apparatus for producing washable absorbent garments having the features of claim 13.

The claims form an integral part of the disclosure provided here in relation to the invention.

### Brief description of the drawings

The present invention will now be described in detail with reference to the attached drawings, given purely by way of non-limiting example, wherein:
- Figure 1 is a perspective view of a washable absorbent garment in an open configuration,
- Figure 2 is a plan view of the absorbent garment of figure 1 in the open configuration,
- Figure 3 is a schematic cross-section taken along the line III-III of figure 2,
- Figures 4 and 5 are schematic side views of an apparatus for producing the washable absorbent garment of figures 1-3,
- Figures 6-11 are schematic plan views showing intermediate steps of a method for producing washable absorbent garments, and
- Figure 12 is a schematic side view showing an alternative to the part of the apparatus shown in figure 5.

It will be appreciated that the various figures may not be represented on the same scale. It will also be appreciated that in some figures certain elements or components may not be shown for a better understanding.

### Detailed description

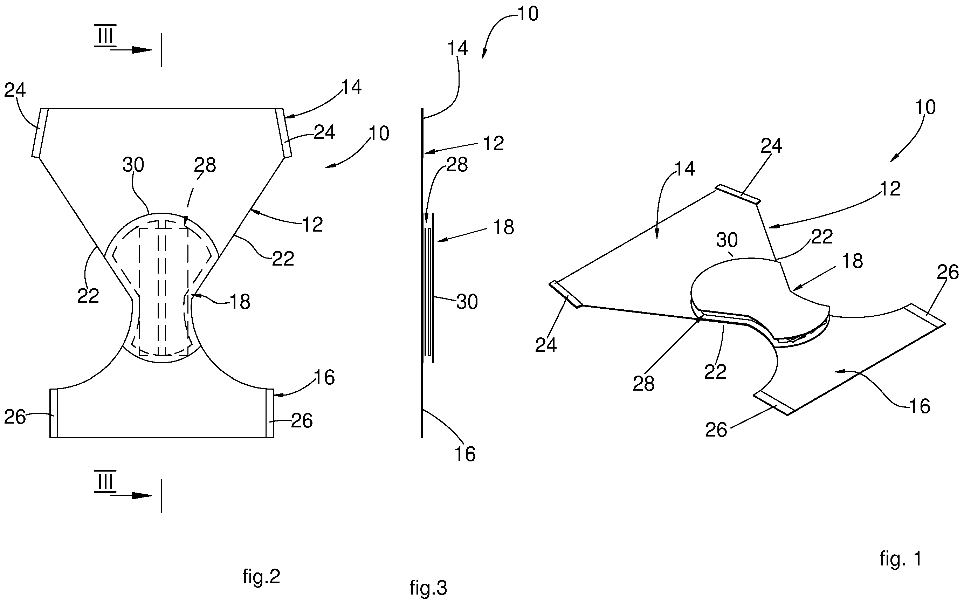

With reference to figures 1-3 the numeral reference 10 indicates a washable absorbent garment.

The washable absorbent garment 10 comprises a back layer 12 of washable fabric forming a pant structure. The back layer 12 has rear and front waist sections 14, 16 and a crotch section 18 intermediate between the rear and front waist sections 14, 16. The rear and front waist sections 14, 16 are wider than the crotch section 18, such that in the extended position of figures 1 and 2 the back layer 12 has substantially an hourglass shape.

The back layer 12 is a fabric formed by woven threads which is washable as ordinary textile garments, e.g. swimsuits, underwear, etc. The back layer 12 may be made of any natural or synthetic material used for producing textile articles, e.g. cotton, Lycra^{®}, nylon, polyester, polypropylene, polyamide, elastane, Econyl^{®} etc. and any mixture thereof. In possible embodiments, the back layer 12 may be an elastic fabric made of a mixture of polyamide and elastane, cotton and elastane e.g. 80% polyamide/cotton and 20% elastane. The back layer 20 may have a weight comprised between 100-200 g/m².

The crotch section 18 of the back layer 12 has two curved side edges 22 shaped to conform to the legs of the user in the configuration in which the washable absorbent garment 10 is worn. The rear and front waist sections 14, 16 of the back layer 12 have respective side edges 24, 26 which are joined to each other such that the washable absorbent garment 10 is closed in a pant-like shape and has two leg openings on opposite sides of the crotch section 18.

The side edges 24, 26 are joined to each other by welding or by glue. Welding may be ultrasonic welding, laser welding, or mechanical welding.

The washable absorbent garment 10 comprises a washable absorbent core 28 permanently fixed in the crotch section 18 of the back layer 12. The washable absorbent core 28 extends only on the crotch section 18 of the back layer 12 and does not extend on the rear and front waist sections 16, 18 of the back layer 12.

The washable absorbent core 28 includes a washable absorbent pad comprising washable superabsorbent fibers. A material suitable for producing the washable absorbent pad 30 may be the one marketed by Technical Absorbents Ltd (UK) under the trade name SAF^{®}. The washable absorbent pad may be formed by needlefelt, thermal bonded, laminated non-woven fibers, formed by polyester, polypropylene and SAF^{®}. The washable absorbent pad may have a weight comprised between 100 and 700 g/m² and a thickness comprised between 1 and 7 mm.

The washable absorbent core 28 may include an acquisition and diffusion layer (ADL) and a barrier film. The washable absorbent pad may be sandwiched between the acquisition and diffusion layer and the barrier film.

The washable absorbent core 28 may have a total weight comprised between 150 and 750 g/m² and a total thickness comprised between 1,5 and 8,5 mm.

The washable absorbent garment 10 comprises a top layer 30 of washable fabric which covers the washable absorbent core 28. The top layer 30 may be made of the same raw materials as the fabric forming the back layer 12. The top layer 30 completely covers the washable absorbent core 28 but does not extend on the rear and front waist sections 16, 18 of the back layer 12.

The top layer 30 and the washable absorbent core 28 are permanently joined to the back layer 12 by welding or by glue.

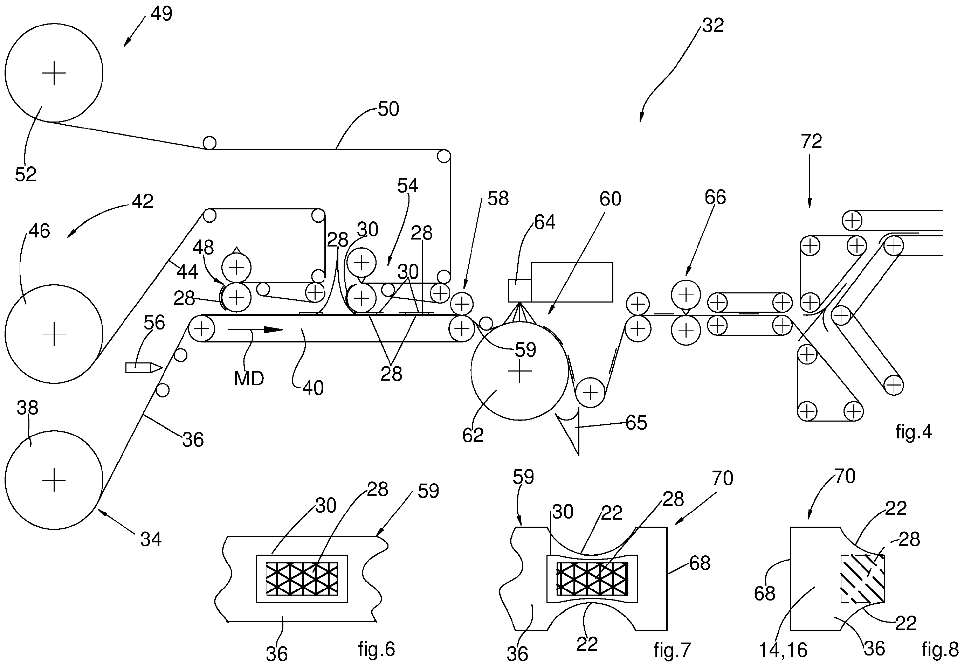

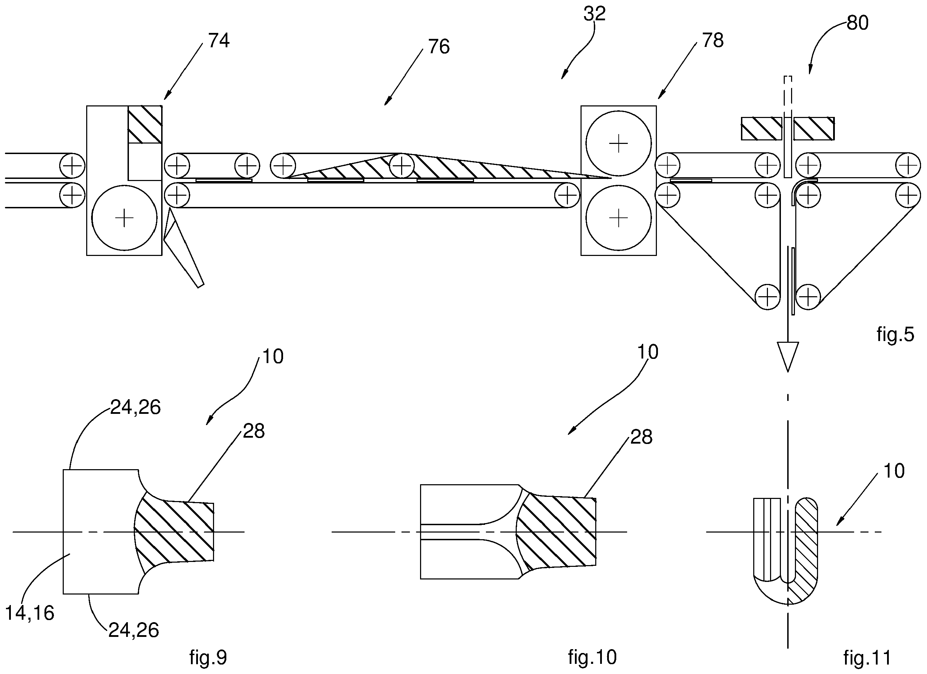

An embodiment of an apparatus for producing the washable absorbent garment 10 of the type illustrated in Figures 1-3 is shown in figures 4 and 5 and is indicated by the reference numeral 32.

With reference to figure 4, the apparatus 32 comprises a first unwinding unit 34 configured for unwinding a first continuous fabric layer 36 from a first reel 38. The first continuous fabric layer 36 is advanced in a machine direction MD on a conveyor 40.

The apparatus 32 comprises a second unwinding unit 42 configured for unwinding a continuous absorbent tape 44 from a second reel 46. The continuous absorbent tape 44 may comprise a web of superabsorbent fibers sandwiched between an acquisition and diffusion layer and a barrier layer. Alternatively, an acquisition and diffusion layer and a barrier layer may be applied in-line on opposite sides of a web of superabsorbent fibers unwound from the second reel 46. The web of superabsorbent fibers may be sandwiched between two soft non-woven layers.

The apparatus 32 comprises a first cut-and-slip unit 48 configured for transversally cutting the continuous absorbent tape 44 to form individual absorbent cores 28. The first cut-and-slip unit 48 is configured for longitudinally spacing from each other the individual absorbent cores 28 and for applying the individual absorbent cores 28 on the first continuous fabric layer 36 in longitudinally spaced positions on the conveyor 40.

The apparatus 32 comprises a third unwinding unit 49 configured for unwinding a second continuous fabric layer 50 from a third reel 52. The apparatus 32 comprises a second cut-and-slip unit 54 configured for transversally cutting the second continuous fabric layer 50 to form individual top layers 30. The second cut-and-slip unit 54 is configured for longitudinally spacing from each other the individual top layers 30 and for applying the individual top layers 30 on respective absorbent cores 28 previously applied on the first continuous fabric layer 36 on the conveyor 40.

The apparatus 32 may comprise a glue dispenser 56 configured for applying a discrete pattern of glue on the first continuous fabric layer 36 for permanently attaching by glue the individual absorbent cores 28 and the individual top layers 30 on the first continuous fabric layer 36.

The apparatus 32 comprises a sealing press 58 located at the end of the conveyor 40, configured for compressing the individual absorbent cores 28 and the individual top layers 30 to the first continuous fabric layer 36. After the compression, the individual absorbent cores 28 and the individual top layers 30 are permanently fixed by glue to the first continuous fabric layer 36 in longitudinally spaced positions to form a continuous garment tape 59.

Alternatively, the apparatus 32 may comprise a welding unit configured for permanently attaching the individual absorbent cores 28 and the individual top layers 30 on the first continuous fabric layer 36. The welding unit may be a ultrasonic or mechanical welding unit.

Figure 6 shows a portion of the continuous garment tape 59 formed by individual absorbent cores 28 and individual top layers 30 fixed to the first continuous fabric layer 36.

The apparatus 32 comprises a cutting unit 60 configured for cutting longitudinal side edges of the continuous garment tape 59 as it advances in the machine direction MD along curved profiles shaped to conform to the legs of the user. The cutting unit 60 comprises an anvil roller 62 and a cutting tool 64 cooperating with the outer surface of the anvil roller 62. The cutting tool 64 may be a laser beam or an ultrasonic horn configured for carrying out a laser or ultrasonic cut of the first continuous fabric layer 36. The cutting tool 64 may also cut simultaneously side edges of the top layers 30.

The cutting tool 64 may be movable transversally to the machine direction to form, in conjunction with the longitudinal movement of the continuous garment tape 59, curved cuts which form curved side edges 22 (figure 7) of the continuous garment tape 59. The portions trimmed from the continuous garment tape 59 are collected in a waste collecting channel 65. The same results could be obtain employing traditional mechanical rotary cutting devices, but with very high limitation with respect to process flexibility and cost.

The apparatus 32 comprises a press unit followed by a transverse cutting unit 66 configured to cut the first continuous fabric layer 36 along transverse cutting lines 68 (figure 7) to form individual blank absorbent garments 70. Figure 7 shows the continuous garment tape 59 after the longitudinal and transverse cutting steps.

The apparatus 32 comprises a transverse folding unit 72 configured for folding the individual blank absorbent garments 70 along respective transverse folding lines, so as to overlap to each other the rear and front waist sections 14, 16 of each blank absorbent garment 70. Figure 8 shows a blank absorbent garment 70 after the transverse folding step.

With reference to figure 5, the apparatus 32 comprises a sealing unit 74 which receives the folded blank absorbent garments 70 from the folding unit 72. With reference to figure 9, the sealing unit 74 is configured for sealing side edges 24, 26 of the rear and front waist sections 14, 16 of the blank absorbent garments 70, to form finished absorbent garments 10. The sealing unit 74 may be an ultrasonic sealing unit configured for carrying out ultrasonic welding of the overlapped side edges 24, 26. Similar results could be obtained with a laser or mechanical sealing unit.

The apparatus 32 may comprise a linear folding unit 76 configured for folding side portions of the finished absorbent garments 10 as shown in figure 10.

The apparatus 32 may comprise a final press unit 78 configured for pressing the folded portions of the finished absorbent garments 10.

The apparatus 32 may comprise a final folding unit 80 configured for carrying out a final folding of the finished absorbent garments 10 as shown in figure 11. After the final folding unit 80 the finished absorbent garments 10 may be sent to a packaging machine.

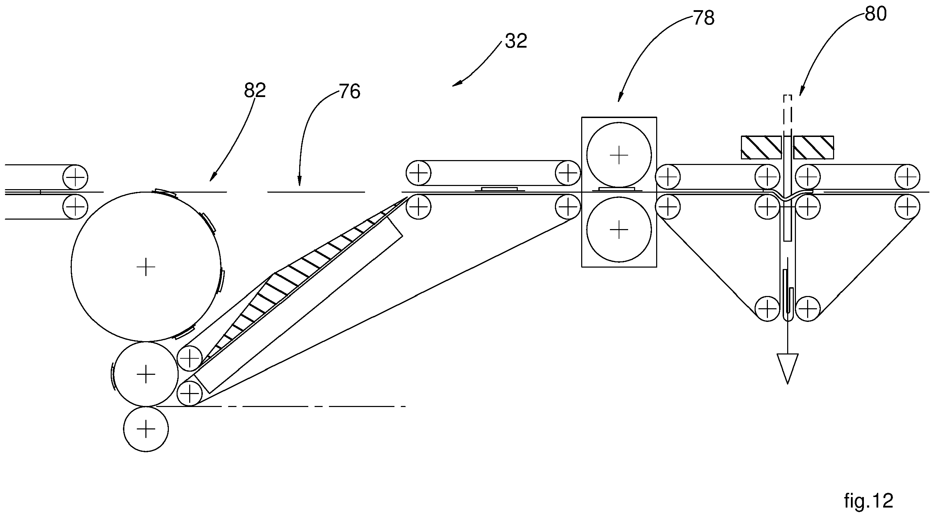

Figure 12 shows an embodiment of the apparatus 32 alternative to the embodiment of figure 5. The elements corresponding to those previously described are indicated by the same reference numbers.

In the embodiment of figure 12 the side edges 24, 26 of the rear and front waist sections 14, 16 of the blank absorbent garments 70 are fixed to each other by glue. The glue dispenser 56 (figure 4) may be configured for applying discrete glue layers also on the portions of the first continuous fabric layer 36 which will form the side edges 24, 26 of the rear and front waist sections 14, 16 of the blank absorbent garments 70. In the embodiment of figure 12 the apparatus 32 comprises a folding and press unit 82 which receives the folded blank absorbent garments 70 from the folding unit 72. The press unit 82 is configured to fold and press the side edges 24, 26 of the rear and front waist sections 14, 16 for glue sealing the side edges 24, 26.

The remaining units 76, 78, 80 of the embodiment of figure 12 are the same as the corresponding units of the embodiment of figure 5.

In operation, the apparatus 32 implements a method for producing washable absorbent garments 10, comprising:
- providing a first continuous fabric layer 36 movable in a longitudinal direction MD,
- applying discrete glue layers on said first continuous fabric layer 36,
- providing a continuous washable absorbent tape 44 containing washable superabsorbent fibers, continuously moving in a longitudinal direction,
- transversely cutting said continuous washable absorbent tape 44 to form individual washable absorbent cores 28,
- spacing from each other in a longitudinal direction said individual washable absorbent cores 28,
- applying said washable absorbent cores 28 on said first continuous fabric layer 36 in longitudinally spaced positions over respective glue layers,
- providing a second continuous fabric layer 50 continuously moving in a longitudinal direction,
- transversely cutting said second continuous fabric layer 50 to form individual top layers 30,
- spacing from each other in a longitudinal direction said individual top layers 30,
- applying said individual top layers 30 over respective washable absorbent cores 28 on said first continuous fabric layer 36, and
- joining said washable absorbent cores 28 and said individual top layers 30 to said first continuous fabric layer 36 by glue to form a continuous washable garment tape (59), and
- transversely cutting said continuous washable garment tape 59 to form individual blank absorbent garments 70.

The method and apparatus according to the present invention may have a production rate up to 200 pieces/1' , which is a huge progress as compared to the production rate of prior art washable absorbent products which is about 1 piece/1'.

Of course, without prejudice to the principle of the invention, the details of construction and the embodiments can be widely varied with respect to those described and illustrated, without thereby departing from the scope of the invention as defined by the claims that follow.

## Claims

1. A washable absorbent garment (10) comprising:
- a back layer (12) of washable fabric forming a pant structure and having rear and front waist sections (14, 16) and a crotch section (18) intermediate between the rear and front waist sections (14, 16), wherein the rear and front waist sections (14, 16) have respective side edges (24, 26) joined to each other by ultrasonic, laser, or mechanical welding or by glue,
- a washable absorbent core (28) including a washable absorbent pad comprising washable superabsorbent fibers, and
- a top layer (30) of washable fabric which covers the washable absorbent core (28),
wherein the top layer (30) and the washable absorbent core (28) are permanently fixed in the crotch section (18) of the back layer (12) by ultrasonic or mechanical welding and/or by glue.

2. The washable absorbent garment of claim 1, wherein the washable absorbent pad has a weight comprised between 100 and 700 g/m².

3. The washable absorbent garment of claim 1 or 2, wherein the washable absorbent pad has a thickness comprised between 1 and 7 mm.

4. The washable absorbent garment of any of the preceding claims, wherein the washable absorbent pad is sandwiched between an acquisition and diffusion layer and a barrier film.

5. The washable absorbent garment of any of the preceding claims, wherein the washable absorbent core (28) has a total weight comprised between 150 and 750 g/m².

6. The washable absorbent garment of any of the preceding claims, wherein the washable absorbent core (28) has a total thickness comprised between 1,5 and 8,5 mm.

7. The washable absorbent garment of any of the preceding claims, wherein the crotch section (18) of the back layer (12) has two curved side edges (22) shaped to conform to the legs of the user in the configuration in which the washable absorbent garment (10) is worn.

8. The washable absorbent garment of any of the preceding claims, wherein the washable absorbent core (28) and the top layer (30) extend only on the crotch section (18) of the back layer (12) and do not extend on the rear and front waist sections (16, 18) of the back layer (12).

9. A method for producing washable absorbent garments (10), comprising:
- providing a first continuous fabric layer (36) movable in a longitudinal direction (MD),
- providing a continuous washable absorbent tape (44) containing washable superabsorbent fibers, continuously moving in a longitudinal direction,
- transversely cutting said continuous washable absorbent tape (44) to form individual washable absorbent cores (28),
- spacing from each other in a longitudinal direction said individual washable absorbent cores (28),
- applying said washable absorbent cores (28) on said first continuous fabric layer (36) in longitudinally spaced positions over respective glue layers,
- providing a second continuous fabric layer (50) continuously moving in a longitudinal direction,
- transversely cutting said second continuous fabric layer (50) to form individual top layers (30),
- spacing from each other in a longitudinal direction said individual top layers (30),
- applying said individual top layers (30) over respective washable absorbent cores (28) on said first continuous fabric layer (36),
- permanently attaching said washable absorbent cores (28) and said individual top layers (30) to said first continuous fabric layer (36) by welding and/or by glue to form a continuous washable garment tape (59), and
- transversely cutting said continuous washable garment tape (59) to form individual blank absorbent garments (70).

10. The method of claim 9, comprising transversely folding said individual blank absorbent garments (70) and overlapping to each other rear and front waist sections (14, 16) of each of said individual blank absorbent garments (70), and joining side edges of said rear and front waist sections (14, 16) by welding and/or glue.

11. The method of claim 9 or claim 10, comprising cutting opposite side edges of said continuous washable garment tape (59) along shaped profiles while advancing said continuous washable garment tape (59) in said longitudinal direction.

12. The method of claim 11, wherein said opposite side edges of said continuous washable garment tape (59) are cut by a mechanical, laser or ultrasonic cutting tool (64).

13. An apparatus for producing washable absorbent garments (10), comprising:
- a first unwinding unit (34) configured for unwinding a first continuous fabric layer (36) from a first reel (38),
- a second unwinding unit (42) configured for unwinding a continuous washable absorbent tape (44) from a second reel (46),
- a first cut-and-slip unit (48) configured for transversally cutting the continuous washable absorbent tape (44) to form individual washable absorbent cores (28), wherein the first cut-and-slip unit (48) is configured for longitudinally spacing from each other the individual absorbent cores (28) and for applying the individual absorbent cores (28) on the first continuous fabric layer (36) in longitudinally spaced positions,
- a third unwinding unit (49) configured for unwinding a second continuous fabric layer (50) from a third reel (52),
- a second cut-and-slip unit (54) configured for transversally cutting the second continuous fabric layer (50) to form individual top layers (30), wherein the second cut-and-slip unit (54) is configured for longitudinally spacing from each other the individual top layers (30) and for applying the individual top layers (30) on respective absorbent cores (28) previously applied on the first continuous fabric layer (36),
- an ultrasonic welding unit or a glue dispenser (56) configured for permanently attaching by ultrasonic welding or by glue the individual absorbent cores (28) and the individual top layers (30) on the first continuous fabric layer (36).

14. The apparatus of claim 13, comprising a side cutting unit (60) configured for cutting longitudinal side edges of a continuous garment tape (59) advancing in a longitudinal direction and a transverse cutting unit (66) configured to cut the continuous garment tape (59) along transverse cutting lines (68) to form individual blank absorbent garments (70).

15. The apparatus of claim 14, comprising a transverse folding unit (72) configured for folding the individual blank absorbent garments (70) along respective transverse folding lines, and a sealing unit (74) configured for sealing side edges (24, 26) of the folded blank absorbent garments (70).
